# EUROPEAN PATENT SPECIFICATION

(11) **EP 3 342 399 B1**
(45) Date of publication and mention of the grant of the patent: **31.08.2022**
(21) Application number: 17020595.9
(22) Date of filing: 31.12.2017
(51) Int. Cl.: A61K 9/16, A61K 9/50, A61K 9/20, A61K 31/496

(54) **PHARMACEUTICAL COMPOSITIONS COMPRISING POSACONAZOLE AND MANUFACTURING METHOD**
PHARMAZEUTISCHE ZUSAMMENSETZUNGEN MIT POSACONAZOL UND HERSTELLUNGSVERFAHREN
COMPOSITIONS PHARMACEUTIQUES COMPRENANT DU POSACONAZOLE ET PROCÉDÉ DE FABRICATION

(30) Priority: 31.12.2016 TR 201620462
(43) Date of publication of application: 04.07.2018
(73) Proprietor: Abdi Ibrahim Ilac Sanayi ve Ticaret A.S., 34500 Esenyurt/Istanbul (TR)
(72) Inventor: Bulent, Kurt, Esenyurt - Istanbul (TR); Kumar, Udaya Dude, Esenyurt - Istanbul (TR)
(74) Representative: Sevinç, Erkan

(56) References cited:
- EP-A1- 1 852 100
- EP-A1- 3 210 599
- EP-A2- 2 285 351
- WO-A1-2017/025292
- WO-A1-2017/032908
- US-A1- 2015 231 081

## Description

### Technical Field of Invention

The present invention relates to a process for preparing pharmaceutical compositions of posaconazole.

### Background of the Invention

Posaconazole is a broad spectrum antifungal agent with thiazole structure. It is represented by Formula 1 below. The chemical name of it is 1-[(1S,2S)-1-Ethyl-2-hydroxypropyl]-4-{4-[4-(4-{2-[(2S,4R)-(1,2,4-triazol-1-ylmetyl)-2-(2,4-difluorophenyl)-oxolan-4-ylmetyl]phenyl}piperazin-1-yl)phenyl]}-2,3-dihydro-1,2,4-triazol-3-one. Like other antifungal drugs, it shows its effect by modifying the fungus cell membrane.

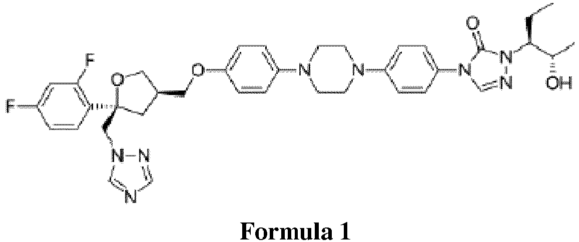

Posaconazole and its synthesis were first disclosed in patent document WO9517407.

Posaconazole shows polymorphism. The best known forms are Form I, Form II, Form Y, Form A and an amorphous form. Details are described in patent documents EP1021439, EP2318004, EP2318396 and WO2015092595.

Posaconazole is a BCS Class II molecule. It has low solubility and high permeability.

Posaconazole shows pH dependent solubility. At low pH its solubility is high. Pharmacokinetic investigations have shown that Posaconazole is absorbed with an average Tmax of 4 to 5 hours. In order to avoid release already in the stomach, enteric dosage forms should be used.

Posaconazole is contained in the marketed product Noxafil, which is available in the form of an oral suspension (40 mg/ml), a concentrated solution for infusion (300 mg/ml) and a tablet (100 mg/tablet).

EP2861213 A1 discloses a composition comprising crystalline posaconazole and one or more surfactants in liquid dosage forms, wherein one of the surfactants is ethoxylated hydrogenated castor oil.

EP2040675 A1 discloses nanoparticulate posaconazole and parenteral compositions comprising posaconazole particles and at least one surface stabilizer.

In the state of the art, the production of tablets containing posaconazole is mentioned. EP2285351 A2 discloses a manufacturing method using hydroxypropyl methyl cellulose acetate succinate (HPMCAS) and posaconazole in a ratio of between 1:1 and 4:1. HPMCAS and posaconazole are mixed in the dry state and then heated to produce a melt. A solid composition is obtained after melt extrusion and cooling.

US2015231081 discloses a delayed release composition comprising posaconazole that is dissolved or dispersed in a polymer which is not a derivative of hydroxypropyl methyl cellulose and a manufacturing method by hot-melt technology.

Non-standard processes like hot-melt extrusion pose complexity in scale up of the process to commercial batch sizes. In addition, process optimization is a relatively difficult task considering the multiple variables involved during the manufacturing process.

EP0954288 B1 discloses a pharmaceutical composition comprising posaconazole and a polymer selected from povidone or crospovidone.

EP0914127 B1 discloses a pharmaceutical composition comprising posaconazole, at least one non-ionic surfactant and one diluent.

If posaconazole is solubilized in a solvent and then mixed with a polymer, solvent evaporation will lead to hardening of the matrix, which in turn can cause trapping of solvent in the matrix. Lowering of the solvent content to the required levels may then not be possible, which, among other things, creates difficulties in the subsequent production steps, such as compressing to tablets.

Considering these problems and disadvantages in the state of the art, there is a need for a new posaconazole formulation and for a new method for producing a pharmaceutical composition containing posaconazole.

There is in particular a need for a posaconazole formulation which is stable during storage, which, after administration, does not release significant amounts of posaconazole in the stomach, but rapidly releases posaconazole once the formulation reaches the small intestine and which shows reduced or no food effect.

Moreover, there is a need for a robust process for preparing a posaconazole formulation which is easy to implement and does not require special equipment, in particular a process which allows for the efficient removal of solvent.

### Description of the Invention

An objective of the present invention is to obtain a formulation comprising posaconazole that solves the aforementioned problems. A further objective is the provision of a method that overcomes the above discussed problems of known methods.

In one aspect, the invention provides a process for preparing enteric-coated granules comprising posaconazole, according to claim 1.

In a further aspect, it is provided (not claimed) a compressed dosage form which comprises coated granules and at least one pharmaceutically acceptable excipient. The compressed dosage form has enteric properties.

Such compressed dosage forms may be obtained by compressing a mixture of enteric coated granules which comprise drug polymer matrix in the form of granules comprising posaconazole, a polymer and at least one pharmaceutical excipient.

Compressed dosage forms are in particular tablets.

A posaconazole formulation obtained by the process according to the present invention is stable during storage.

After administration, the formulation does not release significant amounts of posaconazole in the stomach, but rapidly releases posaconazole once the formulation reaches the small intestine. The formulation preferably shows a reduced or no food effect.

The present invention provides a process for preparing enteric-coated granules which comprises the following steps:
i. Preparing a solution by dissolving posaconazole in a solvent;
ii. Preparing a spraying liquid by addition of hydroxypropylmethylcellulose acetate succinate (HPMCAS) to the solution obtained in (i);
iii. Providing granules of a least one excipient by wet granulation of at least one filler, at least one binder and a solvent;
iv. Top-spraying the liquid obtained in (ii) onto excipient granules of (iii);
v. Drying the drug polymer matrix in the form of granules obtained in (iv) and optionally sieving.

In one aspect, the then obtained enteric coated granules are further treated by a compaction or slugging process to increase the bulk density and the flow attributes. Enteric-coated granules, preferably after the above step, will be mixed with at least one pharmaceutically acceptable excipient and compressed to form a compressed enteric pharmaceutical formulation.

The drug-polymer liquid is sprayed onto granules obtained from wet granulation of at least one filler and at least one binder using aqueous or non-aqueous or a mixture of solvent. Said filler has a capability to retain moisture, wherein the ratio of filler to solvent is in a range of 1:1 to 3:1 in the excipient granule; and then mixture is mixed.

Another objective of the invention is to develop a production method with appropriate quality properties that is simpler, commercially feasible and reproducible in a shorter time than the processes known in the art using hot-melt extrusion techniques, which are complex and difficult to reproduce.

### Detailed Description of the Invention

According to the present invention, a process for preparing pharmaceutical formulations comprising posoconazole is provided according to the claims.

In the present invention, the solid oral pharmaceutical composition may be in tablet, caplet, mini-tablet, granules, capsule, tablet in capsule dosage forms and is not limited thereto. In the present invention, the dosage form is an enteric dosage form, more preferably an enteric tablet.

The term "enteric dosage form" refers to a dosage form that carries enteric properties, namely that is resistant to stomach medium and releases the active ingredient in the intestines. Thus, the pharmaceutical composition containing Posaconazole is prevented from being affected by gastric fluids and irritating the stomach mucosa.

The term "Posaconazole" refers to posaconazole and its pharmaceutically acceptable salts, solvates, hydrates and enantiomers, polymorphs or combinations thereof. In the present invention amorphous or crystalline forms of posaconazole are preferred. The amorphous form is particularly preferred.

The terms "formulation" and "composition" are used interchangeable.

In the present invention, a pharmaceutical composition comprising posaconazole and pharmaceutically acceptable excipients is described. A pharmaceutical composition typically comprises at least one of a polymer, a disintegrant, a binder, a filler, a glidant, a lubricant or combinations thereof. Preferably, the composition comprises at least one polymer, at least one disintegrant, at least one binder, at least one filler, at least one glidant and at least one lubricant.

In the present invention, a liquid comprising a solution of posaconazole dissolved in a solvent and a polymer providing solubility enhancing and delayed-release properties is obtained. This dispersion is used as "spray granulation liquid" in a spray granulation process. That is to say that "spray granulation liquid" refers to a mixture comprising at least one polymer, at least one solvent and posaconazole.

The "solvent" is selected from water, organic solvents like ethanol, methanol, isopropanol, acetone, dichloromethane, dimethylsulphoxide or combinations thereof and mixtures of water and one or more organic solvent. Preferably, the solvent is dichloromethane.

The "polymer providing solubility enhancing and delayed-release properties" refers to hydroxypropylmethylcellulose acetate succinate (HPMCAS).

The inventors contemplate that HMPCAS acts as a delayed-release agent, providing for enteric properties, but at the same time also acts as a solubility enhancing agent for posaconazole. In other words, HMPCAS is a solubility enhancing and delayed-release agent.

In the market, HPMCAS is available as 3 different types, namely L grade, M grade and H grade. These grades can be cohesive fine powder -"F"; or free-flowing granules - "G ."It is surprisingly found that the food effect associated with known dosage forms containing posaconazole is minimized when HPMCAS is used in a product produced according to the present invention. HPMCAS can be M grade, L grade, H grade or a mixture thereof.

The manufacturing process according to the present invention for preparing enteric-coated granules comprises the following steps:
i. Preparing a solution by dissolving posaconazole in a solvent;
ii. Preparing a spraying liquid by addition of HPMCAS to the solution obtained in (i);
iii. Providing granules of a least one excipient by wet granulation of at least one filler, at least one binder and a solvent;
iv. Top-spraying the liquid obtained in (ii) onto excipient granules of (iii);
v. Drying the drug-polymer matrix in the form of granules obtained in (iv) and optionally sieving.

In step (i), a solution of posaconazole dissolved in a solvent is obtained.

In step (ii) a liquid is prepared by combining this solution with HMPCAS . The obtained drug-polymer liquid is sprayed onto the at least one pharmaceutically acceptable excipient which is presented in granular form.

The top-spray granulation method is used.

The granular form of the excipient is manufactured using a wet granulation process. This involves wet granulation of at least one with a binder and a solvent.

The obtained drug-polymer matrix in the form of granules is coated with at least one enteric polymer after step (v) by spray-granulation method. Preferably, the enteric coating polymer is HMPCAS.

According to a further aspect of the invention, the then obtained enteric coated granules are further treated by a compaction or slugging process to increase the bulk density and the flow attributes.

Enteric-coated granules, preferably after the above step, will be mixed with at least one pharmaceutically acceptable excipient and compressed to form a compressed enteric pharmaceutical formulation.

The objective of the present invention is to produce an enteric pharmaceutical composition comprising posaconazole wherein it comprises the following production steps, according to claim 1:
i. Obtaining a solution by dissolving posaconazole in a solvent;
ii. Obtaining a liquid by addition of HPMCAS to the solution obtained in (i);
iii. Granulating the at least one excipient to obtain excipient granules using a wet granulation process;
iv. Top-spraying the liquid obtained in (ii) onto granules of (iii) to produce granules with a drug-polymer matrix;
v. Drying the granules obtained in (iv) and optionally sieving,
vi Coating granules obtained in (v) with at least one enteric polymer by spray-granulation method to obtain enteric coated granules.
vii. Processing the granules obtained in (vi) with or without at least one pharmaceutically acceptable excipients using dry granulation;
viii. Adding at least one pharmaceutically acceptable excipient to granules obtained in (vii), and then mixing and;
ix. compressing the mixture to tablets.

The granular excipient mentioned in step (iii) optionally can be dried.

The obtained enteric coated granules after step (vi) are further treated by a compaction or slugging process to increase the bulk density and the flow attributes.

Enteric coated granules, preferably after the above step, will be mixed with at least one pharmaceutically acceptable excipient and compressed to form a compressed enteric pharmaceutical formulation. If tablets which are compressed after dry granulation are obtained, tablets are optionally coated with a functional or a non-functional coating.

According to the invention, it has been observed that when a top-spray granulation liquid is processed alone, it is not possible to carry out a granulation due to the fact that drug-polymer matrix material is held in the walls of the equipment. With the present invention, excipient granules are top-sprayed with top-spray granulation liquid, so the drug-polymer matrix is not held in the walls of equipment.

Another objective of the present invention is to remove the solvent comprised in top-spray granulation liquid from the granules. The increase in surface area achieved by this production method facilitates removal of the solvent.

In addition, the increase in surface area also has a positive impact on the dissolution rate of the pharmaceutical formulation finally obtained.

The term "wet granulation" refers to wet granulation carried out using at least one filler that has a capability to retain moisture, at least one binder and a solvent.

In the present invention, the solvent used for the wet granulation is water or an organic solvent or mixtures thereof. Preferably, it is water.

The term "filler that has a capability to retain moisture" as used in the wet granulation refers to at least one of starch (potato starch, wheat starch, corn starch, rice starch, including pregelatinized corn starch), lactose, mannitol, microcrystalline cellulose, cellulose and one of the cellulose derivatives or combinations thereof and is not limited thereto. Preferably the filler is corn starch.

The term "binder" as used in the wet granulation refers to hydroxypropylmethylcellulose, hydroxymethylcellulose, hydroxypropylcellulose, micro-crystalline cellulose, starch (corn starch and pregelatinized starch is also included), gelatin, sugars (sucrose, glucose, dextrose, lactose and sorbitol), beeswax, polyethylene glycol, natural and synthetic gums (acacia, gum targacanth, sodium alginate, celluloses) and synthetic polymers like polymethacrylates and polyvinylpyrrolidone (povidone), ethylcellulose, hydroxyethylcellulose, polyethylene oxide or combinations thereof or similar and is not limited thereto. Preferably it is hydroxypropylmethylcellulose.

The term "dry granulation" refers to making granules from active ingredient with or without at least one pharmaceutically acceptable excipient without wetting and subsequent drying processes. In the formulation of the present invention, dry granulation can be obtained by "roller compaction" or "slugging" methods.

In the present invention, if roller-compaction is used, it comprises the following production steps:
a) dried the granules which comprise a drug polymer matrix comprising posaconazole, at least one polymer and at least one pharmaceutical excipient are compacted with or without at least one pharmaceutical excipient by using a roller compactor;
b) the obtained compacts from (a) are milled using suitable milling equipment to get granules and the granules are mixed with at least one pharmaceutical excipient and the mixture is compressed to tablets.

In the present invention, if slugging is used, it comprises the following production steps:
a) dried the granules which comprise a drug polymer matrix comprising posaconazole, at least one polymer and at least one pharmaceutical excipient are sieved and then mixed with at least one pharmaceutical excipient and compressed in the form of slugs;
b) the obtained slugs are milled for obtaining granules and the granules are mixed with at least one pharmaceutical excipient and the mixture is compressed to tablets.

For the formulations of the present invention, pharmaceutically acceptable excipients can be selected, without limitation, from polymers, fillers (diluents), disintegrants, glidants, binders, lubricants and combinations thereof.

The term "filler" refers to, without limitation, at least one of mannitol, lactose, microcrystalline cellulose, calcium carbonate, calcium sulfate, magnesium carbonate, magnesium oxide maltodextrin, maltose, sodium chloride, starch or modified starches (potato starch, wheat starch, corn starch) rice starch, pregelatinized corn starch, sugar, sucrose, sorbitol or combinations thereof. The term "disintegrant" suitable for the formulation of the present invention refers to, without limitation, at least one of croscarmellose sodium, starch, low substituted hydroxypropyl cellulose, crospovidone, alginic acid, sodium alginate, sodium starch glycolate or combinations thereof.

The term "glidant" suitable for the formulation of the present invention wherein it is used to improve followability refers to at least one of colloidal silicon dioxide, aluminum silicate, magnesium silicate, cellulose powder, talc, tribasic calcium phosphate or combinations thereof and is not limited thereto.

The "binder" that is used to provide tablet compressibility refers to, without limitation, at least one of hydroxypropylmethylcellulose, low substituted hydroxypropyl cellulose, hydroxymethyl cellulose, hydroxypropyl cellulose, microcrystalline cellulose, starch (corn starch and pregelatinized starch included), gelatin, sugars (sucrose, glucose, dextrose, lactose and sorbitol included), beeswax, polyethylene glycol, natural and synthetic gums (acacia, gum targacanth, sodium alginate, celluloses) and synthetic polymers like polymethacrylates and polyvinylpyrrolidone (povidone), ethylcellulose, hydroxyethylcellulose, polyethylene oxide or combinations thereof. Preferably it is low substituted hydroxypropyl cellulose.

The term "lubricant" refers to, without limitation, at least one of sodium oleate, sodium stearate, stearic acid, sodium stearyl fumarate, calcium stearate, magnesium stearate, sodium lauryl sulfate, magnesium lauryl sulfate, polyethylene glycol, hydrogenated castor oil, glycerin behenate, sodium benzoate, talc or combinations thereof.

In an embodiment of the invention, a production method for a pharmaceutical formulation containing posaconazole comprises the following steps, according to claim 1:
i. Obtaining a solution by dissolving posaconazole in a solvent;
ii. Obtaining a spraying liquid by addition of HPMCAS to the solution obtained in (i);
iii. Granulating the at least one excipient to obtain excipient granules using a wet granulation process;
iv. Top-Spraying the liquid obtained in (ii) onto granules of (iii) to produce granules with a drug-polymer matrix;
v. Drying the granules obtained in (iv) and optionally sieving;
vi. Processing granules with or without pharmaceutically acceptable excipients using a dry granulation method;
vii. Adding at least one pharmaceutically acceptable excipient to granules obtained in (vi), and then mixing and;
viii. Compressing tablets.

In an embodiment of the invention, a production method for a pharmaceutical formulation containing posaconazole comprises the following steps, according to claim 1:
i. Obtaining a solution by dissolving posaconazole in a solvent;
ii. Obtaining a liquid by addition of HPMCAS to the solution obtained in (i);
iii. Granulating the at least one excipient to obtain excipient granules using a wet granulation process;
iv. Top-spraying the liquid obtained in (ii) onto the granules obtained in (iii);
v. Drying the granules obtained in (iv);
vi. Compacting granules with or without at least one pharmaceutical excipient by using a roller compactor;
vii. Milling compacts obtained in (vi) using suitable milling equipment to get granules;
viii. Adding at least one pharmaceutically acceptable excipient to granules obtained in (vii), and then mixing and;
ix. Compressing tablets.

The obtained drug-polymer matrix in the form of granules is coated with at least one polymer after step (v). The obtained enteric coated granules are then further treated by a compaction or slugging process to increase the bulk density and the flow attributes.

Enteric coated granules, preferably after the above step, will be mixed with at least one pharmaceutically acceptable excipient and compressed to form a compressed enteric pharmaceutical formulation.

In an embodiment of the invention, a production method of pharmaceutical formulation comprising posaconazole comprises the following steps, according to claim 1:
i. Obtaining a solution by dissolving posaconazole in a solvent;
ii. Obtaining a liquid by addition of HPMCAS to the solution obtained in (i);
iii. Granulating the at least one excipient to obtain excipient granules using a wet granulation process;
iv. Top-spraying the liquid obtained in (ii) onto the granules obtained in (iii);
v. Drying the granules obtained in (iv) and optionally sieving;
vi. Compressing granules in the form of slugs with or without at least one pharmaceutical excipient;
vii. Milling the slugs from (vi) using suitable milling equipment to get granules,
viii. Adding at least one pharmaceutically acceptable excipient to granules obtained in (vii), and then mixing and;
ix. Compressing tablets.

The obtained drug-polymer matrix in the form of granules is coated with at least one polymer after step (v), then the obtained enteric coated granules are further treated by a compaction or slugging process to increase the bulk density and the flow attributes.

Enteric coated granules, preferably after the above step, will be mixed with at least one pharmaceutically acceptable excipient and compressed to form a compressed enteric pharmaceutical formulation.

In one embodiment of the present invention, the ratio of posaconazole to polymer is between 1:1 to 1:5, preferably 1:1 to 1:4, in order to achieve a desired dissolution profile for the composition of the present invention. In addition to this, in order to achieve acid resistance in consistent manner, HMPCAS is divided into two fractions, the first fraction is used in a first top-spray granulation and the second fraction is used during a second top-spray granulation for enteric coating. The ratio of the first fraction polymer to second fraction polymer is 4:1, 3:1, 3:1.5 and 3:2, preferably 3:1.

In a specific embodiment of the invention, a production method for a pharmaceutical formulation comprising posaconazole comprises the following steps, according to claim 1:
i. Obtaining a solution by dissolving posaconazole in a solvent;
ii. Adding a first part of HPMCAS to the solution obtained in (i) to get a first liquid;
iii. Granulating the at least one excipient to obtain excipient granules using a wet granulation process;
iv. Top-spraying the first liquid obtained in (ii) onto the excipient granules obtained in (iii);
v. Drying the granules obtained in (iv) and optionally sieving;
vi. Mixing a second part of HPMCAS with a solvent to obtain a second liquid;
vii. Spraying the second liquid obtained in (vi) onto the granules obtained in (v);
viii. Drying the granules obtained in (vii);
ix. Compacting the granules obtained in (viii) by using a roller compactor;
x. Milling the obtained compacts and then mixing the obtained granules with at least one pharmaceutical excipient selected from a filler, a disintegrant, a glidant, a lubricant, a binder or a mixture thereof;
xi. Compressing tablets and optionally coating the tablets;
wherein ratio of first part of HPMCAS to second part of HPMCAS is 3:1 and the solvent in step (i) is dichloromethane and the solvent in step (vi) is mixture of ethanol and water.

In another specific embodiment of the invention, a production method for a pharmaceutical formulation comprising posaconazole is provided which comprises the following steps, according to claim 1:
i. Obtaining a solution by dissolving posaconazole in a solvent,
ii. Adding a first part of HPMCAS to the solution obtained in (i) to get a first liquid;
iii. Granulating the at least one excipient to obtain granules using a wet granulation process;
iv. Top-spraying the first liquid obtained in (ii) onto the granules obtained in (iii);
v. Drying the granules obtained in (iv) and sieving;
vi. Mixing a second part of HPMCAS with a solvent to obtain a second liquid;
vii. Spraying the second liquid obtained in (vi) onto the granules obtained in (v);
viii. Drying the granules obtained in (vii) and sieving,
ix. Mixing the granules with at least one excipient selected from a filler, a disintegrant, a glidant, a lubricant, a binder or mixtures thereof and compressing the mixture to slugs;
x. Milling the obtained slugs and then mixing the granules obtained with at least one pharmaceutical excipient selected from a filler, a disintegrant, a glidant, a lubricant, a binder or mixtures thereof;
xi. Compressing tablets and optionally coating the tablets;
wherein ratio of the first part of HPMCAS to the second part of HPMCAS is 3:1 and the solvent in step (i) is dichloromethane and the solvent in step (vi) is mixture of ethanol and water.

In specific embodiment of the invention, a production method for a pharmaceutical formulation comprising posaconazole comprises the following steps, according to claim 1:
i. Obtaining a solution by dissolving posaconazole in a solvent;
ii. Adding a first part of HPMCAS to the solution obtained in (i) to get a first liquid;
iii. Granulating the at least one excipient to obtain excipient granules using a wet granulation process;
iv. Top-spraying the first liquid obtained in (ii) onto the granules obtained in (iii);
v. Drying the granules obtained in (iv) and sieving;
vi. Mixing a second part of HPMCAS with a solvent to obtain a second liquid;
vii. Top-spraying the second liquid obtained in (vi) onto the granules obtained in (v);
viii. Drying the granules obtained in (vii);
ix. Compacting the granules obtained in (viii) by using a roller compactor;
x. Milling the obtained compacts and then mixing the granules with lactose, microcrystalline cellulose, croscarmellose sodium, colloidal silicon dioxide, hydroxypropylcellulose and magnesium stearate;
xi. Compressing tablets and optionally coating the tablets;
wherein the ratio of the first part of HPMCAS to the second part of HPMCAS is 3:1 and the solvent in step (i) is dichloromethane and the solvent in step (vi) is a mixture of ethanol and water.

In another specific embodiment of the invention, a production method for a pharmaceutical formulation comprising posaconazole comprises the following steps, according to claim 1:
i. Obtaining a solution by dissolving posaconazole in a solvent;
ii. Adding a first part of HPMCAS to the solution obtained in (i) to get a first liquid;
iii. Granulating the at least one excipient to obtain granules using a wet granulation process;
iv. Top-spraying the first liquid obtained in (ii) to the granules obtained in (iii);
v. Drying the granules obtained in (iv) and sieving;
vi. Mixing a second part of HPMCAS with a solvent to obtain a second liquid;
vii. Top-spraying the second liquid obtained in (vi) onto the granules obtained in (v);
viii. Drying the granules obtained in (vii) and sieving;
ix. Compressing slug with lactose, microcrystalline cellulose, croscarmellose sodium, colloidal silicon dioxide, hydroxypropylcellulose and magnesium stearate,
x. Milling the obtained slugs and then mixing the granules obtained with croscarmellose sodium;
xi. Compressing tablets and optionally coating the tablets;
wherein ratio of the first part of HPMCAS to the second part of HPMCAS is 3:1 and the solvent in step (i) is dichloromethane and the solvent in step (vi) is a mixture of ethanol and water.

Present invention is described in more detail with reference to the following examples. The examples are not intended to limit the scope of the invention, but should be considered in light of the description given above.

### Example 1:

| **Ingredients** | **Unit formula (mg)** |
|---|---|
| Posaconazole | 100,00 |
| HPMCAS | 400,00 |
| Hydroxypropylmethyl cellulose (HPMC) | 2,00 |
| Corn starch | 200,00 |
| Lactose monohydrate | 100,00 |
| Microcrystalline cellulose | 58,00 |
| Croscarmellose sodium | 10,00 |
| L-HPC | 20,00 |
| Aerosil | 5,00 |
| Magnesium stearate | 5,00 |
| Dichloromethane^{∗} | q.s |
| Ethanol^{∗} | q.s |
| Water^{∗} | q.s |
| **Tablet Weight** | **900 mg** |

| | |
|---|---|
| ^{∗} is not found in final product | |

Steps of production method is described below:
i. Obtaining a solution by dissolving posaconazole in dichloromethane
ii. Adding a first part of HPMCAS (100 mg) to the solution obtained in (i) to get a first liquid;
iii. Granulating corn starch by using aqueous HPMC
iv. Top-spraying the first liquid obtained in (ii) to the granules obtained in (iii);
v. Drying the granules obtained in (iv) and sieving;
vi. Mixing a second part of HPMCAS (300 mg) with mixture of water and ethanol to obtain a second liquid;
vii. Top-spraying the second liquid obtained in (vi) onto the granules obtained in (v);
viii. Drying the granules obtained in (vii) and sieving;
ix. Compacting the granules obtained in (viii) by using a roller compactor;
x. Milling the obtained compacts and then mixing the granules with lactose, microcrystalline cellulose, croscarmellose sodium, colloidal silicon dioxide, hydroxypropylcellulose and magnesium stearate;
xi. Compressing tablets and optionally coating the tablets;

The tablet can be also produced by slug method which is described above.

Obtained tablets with enteric properties analyzed by dissolution method and conditions. Conditions consists of two stages: acidic stage 2 hours, and buffer stage, pH 6.8 phosphate buffer + 0.37 % Tween 80, 60 minutes, 1000 ml, 75 rpm and the results are shown below in Table-1.

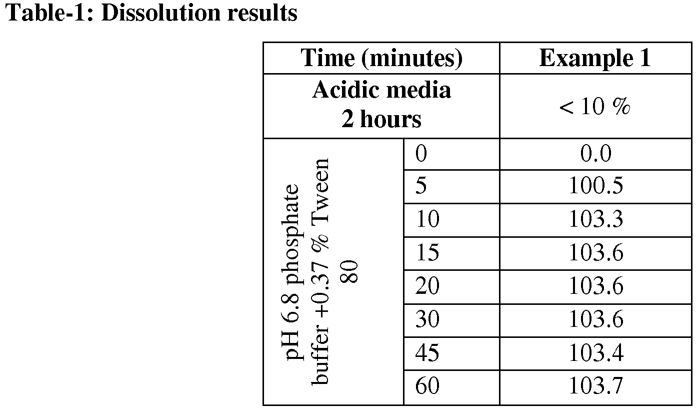

**Comparative Data:** A pharmaceutical tablets which is produced according to this invention (Test 1, "T1") are compared with commercial available reference product (reference, "R") in bioequivalence study under feed and fasting conditions According to results, it was surprisingly found that a pharmaceutical tablet in the present invention shows much minimized food effect.

Table 2 and Table 3 summarize respectively 90% Confidence Intervals for Cmax and AUC of Test 1 (from fasting and feed studies) and Reference product.

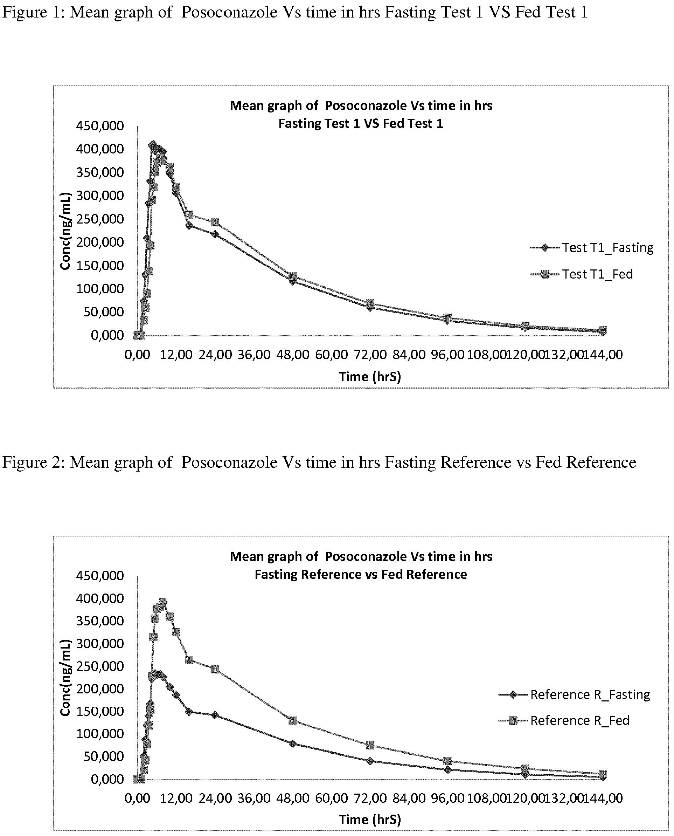

In addition Figure 1 shows posaconazole Vs time in hrs for Test 1 and Figure 2 shows posaconazole Vs time in hrs for Reference under feed conditions.

**Table 2: 90% Confidence Intervals for Cmax and AUC of Test 1 (from fasting study ) vs. Test 1 (from fed study)**

| **_NAME_** | **GM_Fast_T1** | **GM_Fed_T1** | **ratio** | **power** | **inter_cv** | **lower** | **upper** |
|---|---|---|---|---|---|---|---|
| **LAUCinf** | 14299,9900 | 15480,7000 | 92,3730 | 79,1675 | 26,6931 | 79,4936 | 107,3391 |
| **LAUCt** | 13948,4000 | 14981,2400 | 93,1058 | 81,2706 | 25,8684 | 80,4847 | 107,7059 |
| **LCmax** | 454,8918 | 439,7727 | 103,4379 | 91,5275 | 21,5480 | 91,5639 | 116,8518 |

**Table 3: 90% Confidence Intervals for Cmax and AUC of Reference (from fasting study ) vs. Reference (from fed study)**

| **_NAME_** | **GM_Fast_R** | **GM_Fed_R** | **ratio** | **power** | **inter_cv** | **lower** | **upper** |
|---|---|---|---|---|---|---|---|
| **LAUCinf** | 8993,8050 | 15593,5700 | 57,6764 | 62,7194 | 33,5625 | 47,8396 | 69,5358 |
| **LAUCt** | 8706,3160 | 14893,7200 | 58,4563 | 62,9189 | 33,4694 | 48,5103 | 70,4414 |
| **LCmax** | 250,0822 | 432,9848 | 57,7577 | 68,8186 | 30,8581 | 48,6008 | 68,6400 |

## Claims

1. A process for preparing enteric-coated granules which comprises
i. Preparing a solution by dissolving posaconazole in a solvent;
ii. Preparing a spraying liquid by addition of hydroxypropylmethylcellulose acetate succinate (HPMCAS) to the solution obtained in (i);
iii. Providing granules of a least one excipient by wet granulation of at least one filler, at least one binder and a solvent;
iv. Top-spraying the liquid obtained in (ii) onto granules of (iii);
v. Drying the granules obtained in (iv) and optionally sieving.

2. The process according to claim 1, wherein the filler is corn starch.

3. The process according to claim 1, wherein the binder is hydroxypropylmethylcellulose.

4. The process according to any of claims 1 to 3, wherein the granules are provided with an additional enteric coating applied to granules coated with the liquid.

5. The process according to claim 4, wherein the enteric-coated granules are further subjected to dry granulation.

6. The process according to claim 5, wherein the dry granulation is obtained by roller compaction.

7. The process according to claim 6, wherein the process comprises the steps of;
i. Obtaining a solution by dissolving posaconazole in a solvent;
ii. Obtaining a liquid by addition of HPMCAS to the solution obtained in (i);
iii. Granulating the at least one excipient to obtain excipient granules using a wet granulation process;
iv. Top-spraying the liquid obtained in (ii) onto the granules obtained in (iii);
v. Drying the granules obtained in (iv);
vi. Compacting granules with or without at least one pharmaceutical excipient by using a roller compactor;
vii. Milling compacts obtained in (vi) using suitable milling equipment to get granules;
viii. Adding at least one pharmaceutically acceptable excipient to granules obtained in (vii), and then mixing and;
ix. Compressing tablets.

8. The process according to claim 6, wherein the process comprises the steps of;
i. Obtaining a solution by dissolving posaconazole in dichloromethane;
ii. Adding a first part of HPMCAS to the solution obtained in (i) to get a first liquid;
iii. Granulating the at least one excipient to obtain excipient granules using a wet granulation process;
iv. Top-spraying the first liquid obtained in (ii) onto the excipient granules obtained in (iii);
v. Drying the granules obtained in (iv) and optionally sieving;
vi. Mixing a second part of HPMCAS with a mixture of ethanol and water to obtain a second liquid;
vii. Spraying the second liquid obtained in (vi) onto the granules obtained in (v);
viii. Drying the granules obtained in (vii);
ix. Compacting the granules obtained in (viii) by using a roller compactor;
x. Milling the obtained compacts and then mixing the obtained granules with at least one pharmaceutical excipient selected from a filler, a disintegrant, a glidant, a lubricant, a binder or a mixture thereof;
xi. Compressing tablets and optionally coating the tablets; wherein the weight ratio of the first part of HPMCAS to the second part of HPMCAS is 3:1.

9. The process according to claim 6, wherein the process comprises the steps of;
i. Obtaining a solution by dissolving posaconazole in dichloromethane;
ii. Adding a first part of HPMCAS to the solution obtained in (i) to get a first liquid;
iii. Granulating the at least one excipient to obtain excipient granules using a wet granulation process;
iv. Top-spraying the first liquid obtained in (ii) onto the granules obtained in (iii);
v. Drying the granules obtained in (iv) and sieving;
vi. Mixing a second part of HPMCAS with a mixture of ethanol and water to obtain a second liquid;
vii. Top-spraying the second liquid obtained in (vi) onto the granules obtained in (v);
viii. Drying the granules obtained in (vii);
ix. Compacting the granules obtained in (viii) by using a roller compactor;
x. Milling the obtained compacts and then mixing the granules with lactose, microcrystalline cellulose, croscarmellose sodium, colloidal silicon dioxide, hydroxypropylcellulose and magnesium stearate;
xi. Compressing tablets and optionally coating the tablets; wherein the weight ratio of the first part of HPMCAS to the second part of HPMCAS is 3:1.

10. The process according to claim 5, wherein the dry granulation is obtained by slugging method.

11. The process according to claim 10; wherein the process comprises the steps of;
i. Obtaining a solution by dissolving posaconazole in a solvent;
ii. Obtaining a liquid by addition of HPMCAS to the solution obtained in (i);
iii. Granulating the at least one excipient to obtain excipient granules using a wet granulation process;
iv. Top-spraying the liquid obtained in (ii) onto the granules obtained in (iii);
v. Drying the granules obtained in (iv) and optionally sieving;
vi. Compressing granules in the form of slugs with or without at least one pharmaceutical excipient;
vii. Milling the slugs from (vi) using suitable milling equipment to get granules,
viii. Adding at least one pharmaceutically acceptable excipient to granules obtained in (vii), and then mixing and;
ix. Compressing tablets.

12. The process according to claim 10; wherein the process comprises the steps of;
i. Obtaining a solution by dissolving posaconazole in dichloromethane;
ii. Adding a first part of HPMCAS to the solution obtained in (i) to get a first liquid;
iii. Granulating the at least one excipient to obtain granules using a wet granulation process;
iv. Top-spraying the first liquid obtained in (ii) onto the granules obtained in (iii);
v. Drying the granules obtained in (iv) and sieving;
vi. Mixing a second part of HPMCAS with a mixture of ethanol and water to obtain a second liquid;
vii. Spraying the second liquid obtained in (vi) onto the granules obtained in (v);
viii. Drying the granules obtained in (vii) and sieving,
ix. Mixing the granules with at least one excipient selected from a filler, a disintegrant, a glidant, a lubricant, a binder or mixtures thereof and compressing the mixture to slugs;
x. Milling the obtained slugs and then mixing the granules obtained with at least one pharmaceutical excipient selected from a filler, a disintegrant, a glidant, a lubricant, a binder or mixtures thereof;
xi. Compressing tablets and optionally coating the tablets; wherein the weight ratio of the first part of HPMCAS to the second part of HPMCAS is 3:1.

13. The process according to claim 10; wherein the process comprises the steps of;
i. Obtaining a solution by dissolving posaconazole in dichloromethane;
ii. Adding a first part of HPMCAS to the solution obtained in (i) to get a first liquid;
iii. Granulating the at least one excipient to obtain granules using a wet granulation process;
iv. Top-spraying the first liquid obtained in (ii) to the granules obtained in (iii);
v. Drying the granules obtained in (iv) and sieving;
vi. Mixing a second part of HPMCAS with a mixture of ethanol and water to obtain a second liquid;
vii. Top-spraying the second liquid obtained in (vi) onto the granules obtained in (v);
viii. Drying the granules obtained in (vii) and sieving;
ix. Compressing slug with lactose, microcrystalline cellulose, croscarmellose sodium, colloidal silicon dioxide, hydroxypropylcellulose and magnesium stearate,
x. Milling the obtained slugs and then mixing the granules obtained with croscarmellose sodium;
xi. Compressing tablets and optionally coating the tablets; wherein the weight ratio of the first part of HPMCAS to the second part of HPMCAS is 3:1.

## Patentansprüche

1. Verfahren zur Herstellung eines magensaftresistenten Granulats, das die folgenden Schritte umfasst:
i. Herstellen einer Lösung durch Lösen von Posaconazol in einem Lösungsmittel;
ii. Herstellen einer Sprühflüssigkeit durch Zugabe von Hydroxypropylmethylcelluloseacetatsuccinat (HPMCAS) zu der in Schritt (i) erhaltenen Lösung;
iii. Bereitstellen eines Granulats aus mindestens einem Trägerstoff durch Nassgranulation von mindestens einem Füllstoff, mindestens einem Bindemittel und einem Lösungsmittel;
iv. Aufsprühen der in Schritt (ii) erhaltenen Flüssigkeit auf das Granulat von Schritt (iii);
v. Trocknen des in Schritt (iv) erhaltenen Granulats und optional Sieben.

2. Verfahren nach Anspruch 1, wobei es sich bei dem Füllstoff um Maisstärke handelt.

3. Verfahren nach Anspruch 1, wobei es sich bei dem Bindemittel um Hydroxypropylmethylcellulose handelt.

4. Verfahren nach einem der Ansprüche 1 bis 3, wobei das Granulat mit einem zusätzlichen magensaftresistenten Überzug versehen wird, der auf das mit der Flüssigkeit überzogene Granulat aufgebracht wird.

5. Verfahren nach Anspruch 4, wobei das magensaftresistente Granulat ferner einer Trockengranulation unterzogen wird.

6. Verfahren nach Anspruch 5, wobei die Trockengranulation durch Walzenkompaktierung erreicht wird.

7. Verfahren nach Anspruch 6, wobei das Verfahren die folgenden Schritte umfasst:
i. Erhalten einer Lösung durch Lösen von Posaconazol in einem Lösungsmittel;
ii. Erhalten einer Flüssigkeit durch Zugabe von HPMCAS zu der in Schritt (i) erhaltenen Lösung;
iii. Granulation des mindestens einen Trägerstoffs, um mit Hilfe eines Nassgranulationsverfahrens ein Trägerstoffgranulat zu erhalten;
iv. Aufsprühen der in Schritt (ii) erhaltenen Flüssigkeit auf das in Schritt (iii) erhaltene Granulat;
v. Trocknen des in Schritt (iv) erhaltenen Granulats;
vi. Kompaktieren des Granulats mit oder ohne mindestens einen pharmazeutischen Trägerstoff unter Verwendung eines Walzenkompaktierers;
vii. Mahlen der in Schritt (vi) erhaltenen Presslinge unter Verwendung einer geeigneten Mahlanlage zu einem Granulat;
viii. Zugabe mindestens eines pharmazeutisch akzeptablen Trägerstoffs zu dem in Schritt (vii) erhaltenen Granulat und dann Mischen; und
ix. Tablettieren.

8. Verfahren nach Anspruch 6, wobei das Verfahren die folgenden Schritte umfasst:
i. Erhalten einer Lösung durch Lösen von Posaconazol in Dichlormethan;
ii. Zugabe eines ersten Teils von HPMCAS zu der in Schritt (i) erhaltenen Lösung, um eine erste Flüssigkeit zu bekommen;
iii. Granulation des mindestens einen Trägerstoffs, um mit Hilfe eines Nassgranulationsverfahrens ein Trägerstoffgranulat zu erhalten;
iv. Aufsprühen der in Schritt (ii) erhaltenen ersten Flüssigkeit auf das in Schritt (iii) erhaltene Trägerstoffgranulat;
v. Trocknen des in Schritt (iv) erhaltenen Granulats und optional Sieben;
vi. Mischen eines zweiten Teils von HPMCAS mit einer Mischung aus Ethanol und Wasser, um eine zweite Flüssigkeit zu erhalten;
vii. Sprühen der in Schritt (vi) erhaltenen zweiten Flüssigkeit auf das in Schritt (v) erhaltene Granulat;
viii. Trocknen des in Schritt (vii) erhaltenen Granulats;
ix. Kompaktieren des in Schritt (viii) erhaltenen Granulats unter Verwendung eines Walzenkompaktierers;
x. Mahlen der erhaltenen Presslinge und dann Mischen des erhaltenen Granulats mit mindestens einem pharmazeutischen Trägerstoff, der aus einem Füllstoff, einem Zerfallsmittel, einem Gleitmittel, einem Schmiermittel, einem Bindemittel oder einer Mischung davon ausgewählt ist;
xi. Tablettieren und optional Beschichten der Tabletten;
wobei das Gewichtsverhältnis des ersten Teils von HPMCAS zu dem zweiten Teil von HPMCAS 3:1 beträgt.

9. Verfahren nach Anspruch 6, wobei das Verfahren die folgenden Schritte umfasst:
i. Erhalten einer Lösung durch Lösen von Posaconazol in Dichlormethan;
ii. Zugabe eines ersten Teils von HPMCAS zu der in Schritt (i) erhaltenen Lösung, um eine erste Flüssigkeit zu bekommen;
iii. Granulation des mindestens einen Trägerstoffs, um mit Hilfe eines Nassgranulationsverfahrens ein Trägerstoffgranulat zu erhalten;
iv. Aufsprühen der in Schritt (ii) erhaltenen ersten Flüssigkeit auf das in Schritt (iii) erhaltene Granulat;
v. Trocknen des in Schritt (iv) erhaltenen Granulats und Sieben;
vi. Mischen eines zweiten Teils von HPMCAS mit einer Mischung aus Ethanol und Wasser, um eine zweite Flüssigkeit zu erhalten;
vii. Aufsprühen der in Schritt (vi) erhaltenen zweiten Flüssigkeit auf das in Schritt (v) erhaltene Granulat;
viii. Trocknen des in Schritt (vii) erhaltenen Granulats;
ix. Kompaktieren des in Schritt (viii) erhaltenen Granulats unter Verwendung eines Walzenkompaktierers;
x. Mahlen der erhaltenen Presslinge und dann Mischen des Granulats mit Lactose, mikrokristalliner Cellulose, Croscarmellosenatrium, kolloidalem Siliciumdioxid, Hydroxypropylcellulose und Magnesiumstearat;
xi. Tablettieren und optional Beschichten der Tabletten;
wobei das Gewichtsverhältnis des ersten Teils von HPMCAS zu dem zweiten Teil von HPMCAS 3:1 beträgt.

10. Verfahren nach Anspruch 5, wobei die Trockengranulation durch ein Stoßverfahren erreicht wird.

11. Verfahren nach Anspruch 10; wobei das Verfahren die folgenden Schritte umfasst:
i. Erhalten einer Lösung durch Lösen von Posaconazol in einem Lösungsmittel;
ii. Erhalten einer Flüssigkeit durch Zugabe von HPMCAS zu der in Schritt (i) erhaltenen Lösung;
iii. Granulation des mindestens einen Trägerstoffs, um mit Hilfe eines Nassgranulationsverfahrens ein Trägerstoffgranulat zu erhalten;
iv. Aufsprühen der in Schritt (ii) erhaltenen Flüssigkeit auf das in Schritt (iii) erhaltene Granulat;
v. Trocknen des in Schritt (iv) erhaltenen Granulats und optional Sieben;
vi. Komprimieren des Granulats in Form von Pellets mit oder ohne mindestens einen pharmazeutischen Trägerstoff;
vii. Mahlen der Pellets aus Schritt (vi) unter Verwendung einer geeigneten Mahlanlage zu einem Granulat;
viii. Zugabe mindestens eines pharmazeutisch akzeptablen Trägerstoffs zu dem in Schritt (vii) erhaltenen Granulat und dann Mischen; und
ix. Tablettieren.

12. Verfahren nach Anspruch 10; wobei das Verfahren die folgenden Schritte umfasst:
i. Erhalten einer Lösung durch Lösen von Posaconazol in Dichlormethan;
ii. Zugabe eines ersten Teils von HPMCAS zu der in Schritt (i) erhaltenen Lösung, um eine erste Flüssigkeit zu bekommen;
iii. Granulation des mindestens einen Trägerstoffs, um mit Hilfe eines Nassgranulationsverfahrens ein Granulat zu erhalten;
iv. Aufsprühen der in Schritt (ii) erhaltenen ersten Flüssigkeit auf das in Schritt (iii) erhaltene Granulat;
v. Trocknen des in Schritt (iv) erhaltenen Granulats und Sieben;
vi. Mischen eines zweiten Teils von HPMCAS mit einer Mischung aus Ethanol und Wasser, um eine zweite Flüssigkeit zu erhalten;
vii. Sprühen der in Schritt (vi) erhaltenen zweiten Flüssigkeit auf das in Schritt (v) erhaltene Granulat;
viii. Trocknen des in Schritt (vii) erhaltenen Granulats und Sieben;
ix. Mischen des Granulats mit mindestens einem Trägerstoff, der aus einem Füllstoff, einem Zerfallsmittel, einem Gleitmittel, einem Schmiermittel, einem Bindemittel oder Mischungen davon ausgewählt ist, und Komprimieren der Mischung zu Pellets;
x. Mahlen der erhaltenen Pellets und dann Mischen des erhaltenen Granulats mit mindestens einem pharmazeutischen Trägerstoff, der aus einem Füllstoff, einem Zerfallsmittel, einem Gleitmittel, einem Schmiermittel, einem Bindemittel oder Mischungen davon ausgewählt ist;
xi. Tablettieren und optional Beschichten der Tabletten;
wobei das Gewichtsverhältnis des ersten Teils von HPMCAS zu dem zweiten Teil von HPMCAS 3:1 beträgt.

13. Verfahren nach Anspruch 10; wobei das Verfahren die folgenden Schritte umfasst:
i. Erhalten einer Lösung durch Lösen von Posaconazol in Dichlormethan;
ii. Zugabe eines ersten Teils von HPMCAS zu der in Schritt (i) erhaltenen Lösung, um eine erste Flüssigkeit zu bekommen;
iii. Granulation des mindestens einen Trägerstoffs, um mit Hilfe eines Nassgranulationsverfahrens ein Granulat zu erhalten;
iv. Aufsprühen der in Schritt (ii) erhaltenen ersten Flüssigkeit auf das in Schritt (iii) erhaltene Granulat;
v. Trocknen des in Schritt (iv) erhaltenen Granulats und Sieben;
vi. Mischen eines zweiten Teils von HPMCAS mit einer Mischung aus Ethanol und Wasser, um eine zweite Flüssigkeit zu erhalten;
vii. Aufsprühen der in Schritt (vi) erhaltenen zweiten Flüssigkeit auf das in Schritt (v) erhaltene Granulat;
viii. Trocknen des in Schritt (vii) erhaltenen Granulats und Sieben;
ix. Komprimieren der Pellets mit Lactose, mikrokristalliner Cellulose, Croscarmellosenatrium, kolloidalem Siliciumdioxid, Hydroxypropylcellulose und Magnesiumstearat;
x. Mahlen der erhaltenen Pellets und dann Mischen des erhaltenen Granulats mit Croscarmellosenatrium;
xi. Tablettieren und optional Beschichten der Tabletten;
wobei das Gewichtsverhältnis des ersten Teils von HPMCAS zu dem zweiten Teil von HPMCAS 3:1 beträgt.

## Revendications

1. - Procédé de préparation de granulés à enrobage entérique, qui comprend :
i. Préparer une solution par dissolution de posaconazole dans un solvant ;
ii. Préparer un liquide de pulvérisation par addition d'acétate succinate d'hydroxypropylméthylcellulose (HPMCAS) à la solution obtenue en (i) ;
iii. Fournir des granulés d'au moins un excipient par granulation par voie humide d'au moins une charge, d'au moins un liant et d'un solvant ;
iv. Pulvériser par le haut le liquide obtenu en (ii) sur les granulés de (iii) ;
v. Sécher les granulés obtenus en (iv) et facultativement tamiser.

2. - Procédé selon la revendication 1, dans lequel la charge est de l'amidon de maïs.

3. - Procédé selon la revendication 1, dans lequel le liant est l'hydropropylméthylcellulose.

4. - Procédé selon l'une quelconque des revendications 1 à 3, dans lequel les granulés sont munis d'un enrobage entérique supplémentaire appliqué aux granulés enrobés par le liquide.

5. - Procédé selon la revendication 4, dans lequel les granulés à enrobage entérique sont en outre soumis à une granulation par voie sèche.

6. - Procédé selon la revendication 5, dans lequel la granulation par voie sèche est obtenue par compactage au rouleau.

7. - Procédé selon la revendication 6, dans lequel le procédé comprend les étapes consistant à :
i. Obtenir une solution par dissolution de posaconazole dans un solvant ;
ii. Obtenir un liquide par ajout de HPMCAS à la solution obtenue en (i) ;
iii. Granuler ledit au moins un excipient pour obtenir des granulés d'excipient à l'aide d'un procédé de granulation par voie humide ;
iv. Pulvériser par le haut le liquide obtenu en (ii) sur les granulés obtenus en (iii) ;
v. Sécher les granulés obtenus en (iv) ;
vi. Compacter des granulés avec ou sans au moins un excipient pharmaceutique à l'aide d'un rouleau compacteur ;
vii. Broyer les produits de compactage obtenus en (vi) à l'aide d'un équipement de broyage approprié pour obtenir des granulés ;
viii. Ajouter au moins un excipient pharmaceutiquement acceptable aux granulés obtenus en (vii), puis mélanger et ;
ix. Comprimer des comprimés.

8. - Procédé selon la revendication 6, dans lequel le procédé comprend les étapes consistant à :
i. Obtenir une solution par dissolution de posaconazole dans du dichlorométhane ;
ii. Ajouter une première partie de HPMCAS à la solution obtenue en (i) pour obtenir un premier liquide ;
iii. Granuler ledit au moins un excipient pour obtenir des granulés d'excipient à l'aide d'un procédé de granulation par voie humide ;
iv. Pulvériser par le haut le premier liquide obtenu en (ii) sur les granulés d'excipient obtenus en (iii) ;
v. Sécher les granulés obtenus en (iv) et facultativement tamiser ;
vi. Mélanger une seconde partie de HPMCAS avec un mélange d'éthanol et d'eau pour obtenir un second liquide ;
vii. Pulvériser le second liquide obtenu en (vi) sur les granulés obtenus en (v) ;
viii. Sécher les granulés obtenus en (vii) ;
ix. Compacter les granulés obtenus en (viii) à l'aide d'un rouleau compacteur ;
x. Broyer les produits de compactage obtenus, puis mélanger les granulés obtenus avec au moins un excipient pharmaceutique choisi parmi une charge, un délitant, un agent de glissement, un lubrifiant, un liant ou un mélange de ceux-ci ;
xi. Comprimer des comprimés et facultativement enrober les comprimés ;
dans lequel le rapport en poids de la première partie de HPMCAS à la seconde partie de HPMCAS est de 3 :1.

9. - Procédé selon la revendication 6, dans lequel le procédé comprend les étapes consistant à :
i. Obtenir une solution par dissolution de posaconazole dans du dichlorométhane ;
ii. Ajouter une première partie de HPMCAS à la solution obtenue en (i) pour obtenir un premier liquide ;
iii. Granuler ledit au moins un excipient pour obtenir des granulés d'excipient à l'aide d'un procédé de granulation par voie humide ;
iv. Pulvériser par le haut le premier liquide obtenu en (ii) sur les granulés obtenus en (iii) ;
v. Sécher les granulés obtenus en (iv) et tamiser ;
vi. Mélanger une seconde partie de HPMCAS avec un mélange d'éthanol et d'eau pour obtenir un second liquide ;
vii. Pulvériser par le haut le second liquide obtenu en (vi) sur les granulés obtenus en (v) ;
viii. Sécher les granulés obtenus en (vii) ;
ix. Compacter les granulés obtenus en (viii) à l'aide d'un rouleau compacteur ;
x. Broyer les produits de compactage obtenus puis mélanger les granulés avec du lactose, de la cellulose microcristalline, de la croscarmellose sodique, du dioxyde de silicium colloïdal, de l'hydroxypropylcellulose et du stéarate de magnésium ;
xi. Comprimer des comprimés et facultativement enrober les comprimés ;
dans lequel le rapport en poids de la première partie de HPMCAS à la seconde partie de HPMCAS est de 3 :1.

10. - Procédé selon la revendication 5, dans lequel la granulation par voie sèche est obtenue par un procédé par briquetage.

11. - Procédé selon la revendication 10, dans lequel le procédé comprend les étapes consistant à :
i. Obtenir une solution par dissolution de posaconazole dans un solvant ;
ii. Obtenir un liquide par addition de HPMCAS à la solution obtenue en (i) ;
iii. Granuler ledit au moins un excipient pour obtenir des granulés d'excipient à l'aide d'un procédé de granulation par voie humide ;
iv. Pulvériser par le haut le liquide obtenu en (ii) sur les granulés obtenus en (iii) ;
v. Sécher les granulés obtenus en (iv) et facultativement tamiser ;
vi. Comprimer des granulés sous la forme de briquettes avec ou sans au moins un excipient pharmaceutique ;
vii. Broyer les briquettes obtenues en (vi) à l'aide d'un équipement de broyage approprié pour obtenir des granulés ;
viii. Ajouter au moins un excipient pharmaceutiquement acceptable aux granulés obtenus en (vii), puis mélanger et ;
ix. Comprimer des comprimés.

12. - Procédé selon la revendication 10, dans lequel le procédé comprend les étapes consistant à :
i. Obtenir une solution par dissolution de posaconazole dans du dichlorométhane ;
ii. Ajouter une première partie de HPMCAS à la solution obtenue en (i) pour obtenir un premier liquide ;
iii. Granuler ledit au moins un excipient pour obtenir des granulés à l'aide d'un procédé de granulation par voie humide ;
iv. Pulvériser par le haut le premier liquide obtenu en (ii) sur les granulés obtenus en (iii) ;
v. Sécher les granulés obtenus en (iv) et tamiser ;
vi. Mélanger une seconde partie de HPMCAS avec un mélange d'éthanol et d'eau pour obtenir un second liquide ;
vii. Pulvériser le second liquide obtenu en (vi) sur les granulés obtenus en (v) ;
viii. Sécher les granulés obtenus en (vii) et tamiser ;
ix. Mélanger les granulés avec au moins un excipient choisi parmi une charge, un délitant, un agent de glissement, un lubrifiant, un liant ou des mélanges de ceux-ci et comprimer le mélange en briquettes ;
x. Broyer les briquettes obtenues puis mélanger les granulés obtenus avec au moins un excipient pharmaceutique choisi parmi une charge, un délitant, un agent de glissement, un lubrifiant, un liant ou des mélanges de ceux-ci ;
xi. Comprimer des comprimés et facultativement enrober les comprimés ;
dans lequel le rapport en poids de la première partie de HPMCAS à la seconde partie de HPMCAS est de 3 :1.

13. - Procédé selon la revendication 10, dans lequel le procédé comprend les étapes consistant à :
i. Obtenir une solution par dissolution de posaconazole dans du dichlorométhane ;
ii. Ajouter une première partie de HPMCAS à la solution obtenue en (i) pour obtenir un premier liquide ;
iii. Granuler ledit au moins un excipient pour obtenir des granulés à l'aide d'un procédé de granulation par voie humide ;
iv. Pulvériser par le haut le premier liquide obtenu en (ii) sur les granulés obtenus en (iii) ;
v. Sécher les granulés obtenus en (iv) et tamiser ;
vi. Mélanger une seconde partie de HPMCAS avec un mélange d'éthanol et d'eau pour obtenir un second liquide ;
vii. Pulvériser par le haut le second liquide obtenu en (vi) sur les granulés obtenus en (v) ;
viii. Sécher les granulés obtenus en (vii) et tamiser ;
ix. Comprimer des briquettes avec du lactose, de la cellulose microcristalline, de la croscarmellose sodique, du dioxyde de silicium colloïdal, de l'hydroxypropylcellulose et du stéarate de magnésium ;
x. Broyer les briquettes obtenues puis mélanger les granulés obtenus avec de la croscarmellose sodique ;
xi. Comprimer des comprimés et facultativement enrober les comprimés ;
dans lequel le rapport en poids de la première partie de HPMCAS à la seconde partie de HPMCAS est de 3 :1.
